# NEW EUROPEAN PATENT SPECIFICATION

(11) **EP 0 571 543 B2**
(45) Date of publication and mention of the opposition decision: **17.03.1999**
(45) Mention of the grant of the patent: 15.03.1995
(21) Application number: 92907100.9
(22) Date of filing: 30.01.1992
(51) Int. Cl.: A61K 7/08, A61K 7/50, A61K 7/02, C11D 1/94

(54) **COSMETIC COMPOSITIONS**
KOSMETISCHE ZUSAMMENSETZUNGEN
COMPOSITIONS COSMETIQUES

(30) Priority: 12.02.1991 US 654177
(43) Date of publication of application: 01.12.1993
(73) Proprietor: RICHARDSON-VICKS, INC., Shelton, CT 06484 (US)
(72) Inventor: LINARES, Carlos Gabriel, Stanford, CT 06905 (US); DECKNER, George Endel, Trumbull, CT 06611 (US); ST. JOHN, Lucie Anita, Waterbury, CT 06708 (US)
(74) Representative: Clemo, Nicholas Graham
(86) International application number: US9200749
(87) International publication number: WO9213513

(56) References cited:
- EP-A- 67 635
- EP-A- 250 181
- EP-A- 0 250 181
- WO-A-91/11984
- WO-A-91/17237
- US-A- 3 980 769
- US-A- 4 181 634
- US-A- 5 641 479
- Formulation of Johnson's baby shampoo formula n 2384
- McCutcheon's emulsifiers and detergents
- Croda Crothix product information (15 June 1989)
- Fitenex CLEAR & MILD SKIN CLEANSING GEL Product information (May 1990)
- Merck Index 1983, 10th edition, p. 5449

## Description

### TECHNICAL FIELD

The present invention relates to improved cosmetic cleansing compositions, and in particular to foaming cosmetic compositions suitable for cleansing the skin and or the hair and which may be used, for example, in the form of make-up removal facial cleansers, bath foams, shower products, shampoos and the like.

### BACKGROUND OF THE INVENTION

Foaming cosmetic compositions must satisfy a number of criteria including cleansing power, foaming properties and mildness/low irritancy with respect to the skin, hair and the occular mucosae.

Skin is made up of several layers of cells which coat and protect the keratin and collagen fibrous proteins that form the skeleton of its structure. The outermost of these layers, referred to as the stratum corneum, is known to be composed of 25 nm (250 Å)diameter protein bundles surrounded by 8 nm (80 Å) thick bilayers of epidermal lipids and water. Anionic surfactants can penetrate the stratum corneum membrane and, by delipidization (i.e. removal of the lipids from the stratum corneum), destroy its integrity. This destruction of the stratum corneum bilayers can lead to dry rough skin and may eventually permit the surfactant to interact with the viable epidermis, creating irritation.

Ideal cosmetic cleansers should cleanse the skin or hair gently, causing little or no irritation without defatting and or drying the skin and without leaving skin taut after frequent use. Most lathering soaps, liquids and bars fail in this respect.

Certain synthetic surfactants are known to be mild However, a major drawback of most mild synthetic surfactant systems, when formulated for skin cleansing, is poor lather performance compared to the highest bar soap standards (bars which are rich in coconut soap and superfatted). On the other hand, the use of known high sudsing anionic surfactants with lather boosters can yield acceptable lather volume and quality. Unfortunately, however, the highest sudsing anionic surfactants are, in fact, poor in clinical skin mildness. Surfactants that are among the mildest, such as sodium lauryl glyceryl ether sulfonate, (AGS), are marginal in lather. These two facts make the balancing of the surfactant selection and the lather and skin feel benefit a delicate process.

Rather stringent requirements for cosmetic cleansers limit the choice of surface-active agents, and final formulations represent some degree of compromise. Mildness is often obtained at the expense of effective cleansing, or lathering may be sacrificed for either mildness, product stability, or both.

Thus a need exists for foaming cosmetic compositions which will produce a foam which is abundant, stable and of high quality (compactness), which are effective skin and hair cleansers and which are very mild to the skin, hair and occular mucosae.

It has been found that the use of specific polyol alkoxy esters in combination with specific amphoteric surfactants provide cosmetic cleansing compositions with significantly improved dermal mildness benefits and also good physical characteristics such as foaming.

It is therefore an object of the present invention to provide an improved cosmetic cleansing composition which thoroughly cleanses the skin and hair and which is very mild to the skin, hair and occular mucosae.

It is a further object of the present invention to provide an improved cosmetic cleansing composition which is mild and which will produce a foam which is abundant, stable and of high quality, and which effectively cleans skin and hair.

### SUMMARY OF THE INVENTION

The present invention relates to foaming cosmetic compositions suitable for cleansing the skin or hair and which may be used as make-up removers and facial cleansers, bath foams, shower products, shampoos, and the like comprising:
(a) from about 0.1% to about 5.0% by weight on a solids basis of a first amphoteric surfactant of formula I selected from imidazolinium derivatives: 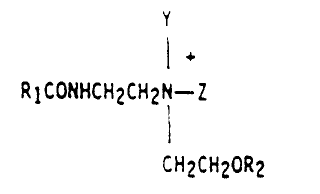 wherein R₁ is C₈-C₂₂ alkyl or alkenyl, R₂ is hydrogen or CH₂COOM, Z is H, CH₂COOM, CH₂CH₂COOM, or CH₂CHOHCH₂SO₃M, Y is H, CH₂COOM, CH₂CH₂COOM, or CH₂CHOHCH₂SO₃M and M is H, alkali metal, alkaline earth metal, ammonium or alkanolammonium;
(b) from about 0.1% to about 5.0% of a polyol alkoxy ester which is a branched polyethylene-glycol compound having molecular weight about 4000 to about 8000; and
(c) from about 60% to about 99.5% water; wherein the ratio of (a):(b) is from about 15:1 to about 1:3 and wherein the cleansing composition has a viscosity of at least about 150 cps (Brookfield RVT, Spindle No TB, 10 rpm, 25°C).

All concentrations and ratios herein are by weight of total composition and all measurements are at 25°C, unless otherwise specified.

### DETAILED DESCRIPTION OF THE INVENTION

The invention relates to a foaming cosmetic composition with superior mildness and excellent lathering characteristics (abundance, quality) and good cleansing ability The foaming cosmetic composition take the form of a viscous liquid, paste or gel which has the advantage that it can be easily and commercially packaged in and dispensed from pump bottles or from tubes by squeezing. Gel form compositions are defined herein as those which have a viscosity (Brookfield RVT, Spindle No. TB, 10 rpm, 25°C) of at least 10,000 cps. Preferred from the viewpoint of flowability are gel compositions having a viscosity in the range from about 10,000 to about 50,000 cps, more preferably from about 10,000 to about 20,000 cps. Viscous liquids or pastes are defined herein as those having a viscosity (same conditions) of at least about 150 cps, preferably at least about 500 cps and less than about 10,000 cps, and more preferably between about 5,000 cps and about 9,000 cps.

### Surfactant

The essential surfactant component of the compositions of the present invention is an amphoteric surfactant selected from imidazolinium surfactants of formula I 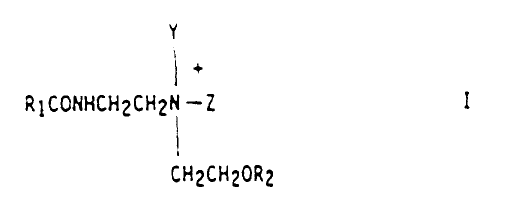 wherein R₁ is C₈-C₂₂ alkyl or alkenyl, R₂ is hydrogen or CH₂COOM, Z is H, CH₂COOM, CH₂CH₂COOM, or CH₂CHOHCH₂SO₃M, Y is H, CH₂COOM, CH₂CH₂COOM, or CH₂CHOHCH₂SO₃M and M is H alkali metal, alkaline earth metal, ammonium or alkanolammonium;

These amphoteric surfactants are present in the compositions of the present invention at a level of from about 0.1% to about 5%, more preferably from about 0.1% to about 3% and most preferably from about 0.1% to about 2.5%, on a solids basis.

Examples of suitable amphoteric surfactants for use herein include compounds in which R₁ is C₈H₁₇-(especially iso-capryl), C₈H₁₉, C₁₁H₂₃ and C₁₂H₂₅ alkyl. Especially preferred are the compounds in which R₁ is C₁₂H₂₅, Z is CH₂CO₂M, Y is CH₂COOM and R₂ is H. These surfactants are all fully described in Miranol Products for Cosmetics and Toiletries Technical and Product Development, Tenth Edition (1987); which is incorporated by reference herein.

It will be understood that a number of commercially-available amphoteric surfactant of this type are manufactured and sold in the form of complexes with anionic surfactants, especially those of the sulfated C₈-C₁₈ alcohol or C₈-C₁₈ acyl glyceride types. More preferably, the compositions comprise the surfactant component along with a premix or complex of the optional amphoteric surfactant and anionic surfactant in an equivalent ratio of about 1:1 in order to provide approximate electroneutrality.

In a preferred embodiment of the present invention, the compositions comprise a second amphoteric surfactant being selected from aminoalkanoates of formula II

R₁NH(CH₂)ₙCO₂M II

iminodialkanoates of formula III

R₁N[(CH₂)ₘCO₂M]₂ III

and mixtures thereof, wherein n and m are numbers from 1 to 4, and R₁ and M are independently selected from the groups specified in I above.

The compositions herein can also comprise other optional surfactant components, notably, anionic surfactants. It is an important feature of the invention, however, that the combined concentration of the first and the optional second amphoteric surfactants is at least about 30% by weight of the total surfactant concentration, this being important from the viewpoint of achieving optimum lathering characteristics. In preferred compositions, the mixture of the first and second amphoteric surfactants comprises at least about 60%, more preferably at least about 75% by weight of the total surfactant composition.

Examples of suitable optional amphoteric surfactants for use herein include salts, especially the triethanolammonium salts and salts of N-lauryl-beta-aminopropionic acid and N-laurylimino-dipropionic acid.

The compositions herein preferably contain from about 1% to about 10% by weight, more preferably from about 1.5% to about 5% by weight of each of the first and second amphoteric surfactants. The weight ratio of first amphoteric surfactant : second amphoteric surfactant is preferably from about 10:1 to about 1:10, more preferably from about 5:1 to about 1:5, and especially from about 3:1 to about 1:3.

A preferred optional surfactant in the compositions herein is an anionic surfactant. This is preferably present in a level of from about 0.1% to about 10%, more preferably from about 1.0% to about 8%, and especially from about 4% to about 6% by weight. Preferred anionic surfactants for inclusion herein, other than the alkyl and acylglyceride sulfates mentioned above, are the fatty acid condensation products of proteins, degraded proteins or amino acids, or mixtures of such condensation products. In highly preferred embodiments, the fatty acid condensation products are selected from:
(i) condensation products of C₈-C₁₂, preferably C₁₀-C₁₈ fatty acids with hydrolysed proteins
(ii) fatty acid sarcosinates derived from C₈-C₂₂, preferably C₁₀-C₁₈ fatty acids, and
(iii) mixtures thereof.

However, the total level of surfactant in the compositions herein should generally lie in the range from about 1% to about 20% by weight, preferably from about 1% to about 15% by weight, and especially from about 2% to about 7% by weight.

### Polyol Alkoxy Ester

The compositions of the present invention also essentially comprise a polyol alkoxy ester which is a branched polyethylene-glycol compound having molecular weight about 4000 to about 8000. These non-ionic compounds are high molecular weight (from abut 4,000 to about 8,000 preferably from about about 6,000 to about 8,000) branched polyethylene glycol compounds. A highly preferred polyol alkoxy ester is available from Croda, Inc. under the trade name Crothix. The polyol alkoxy ester is present in the composition at a level of from about 0.1% to about 5%, preferably from about 0.5% to about 3%, and most preferably from about 1% to about 2%.

The amphoteric surfactant and polyol alkoxy ester are present in a ratio of amphoteric surfactant:polyol alkoxy ester of from about 10:1 to about 1:3, preferably from about 5:1 to about 1:3 and most preferably from about 3:1 to about 1:3.

### Optional Components

The compositions of the invention may also contain additional thickeners at a level preferably from about 0.1% to about 10%, more preferably from about 0.1% to about 5%, and especially from about 0.3% to about 4%. The thickener preferably has a viscosity (1% aqueous solution, 25°C, Brookfield RVT Spindle No TB, 5 rpm) of at least about 4000 cps, more preferably at least about 10,000 cps.

Neutralizing agents suitable for use in neutralizing acidic group containing hydrophilic thickeners herein including sodium hydroxide, potassium hydroxide, ammonium hydroxide, monoethanolamine, diethaniolamine and triethanolamine.

The compositions of the invention can optionally include a hair or skin moisturizer. The preferred level of moisturizer is from about 1% to about 20% by weight. In preferred embodiments, the moisturizer is nonocclusive and is selected from:
1. water-soluble liquid polyols;
2. essential amino acid compounds found naturally occurring in the stratum corneum of the skin; and
3. water-soluble nonocclusives and mixtures thereof.

Some examples of more preferred nonocclusive moisturizers are glycerine, polyethylene glycol, propylene glycol, sorbitol, polyethylene glycol and propylene glycol esters of methyl glucose (e.g. methyl gluxan-20), polyethylene glycol and propylene glycol esters of lanolin alcohol (e.g. Solulan-75), sodium pyrrolidone carboxylic acid, lactic acid, urea, L-proline, guanidine, pyrrolidone and mixtures thereof. Of the above, glycerine is highly preferred.

Examples of other water-soluble nonocclusive moisturizers include water-soluble hexadecyl, myristyl, isodecyl or isopropyl esters of adipic, lactic, oleic, stearic, isostearic, myristic or linoleic acids, as well as many of their corresponding alcohol esters (sodium isostearoyl-2-lactylate, sodium capryl lactylate), hydrolyzed protein and other collagen-derived proteins, aloe vera gel and acetamide MEA.

The compositions of the invention can additionally comprise from about 0.05% to about 5% by weight of cationic or nonionic polymeric hair or skin conditioning agents. Representative classes of polymeric hair or skin conditioning agents include cationic and nonionic polysaccharides; cationic and nonionic homopolymers and copolymers derived from acrylic and/or methacrylic acid; cationic and nonionic cellulose resins; cationic copolymers of dimethyldiallylammonium chloride and acrylic acid; cationic homopolymers of dimethyldiallylammonium chloride; cationic polyalkylene and ethoxypolyalkene imines, and mixtures thereof.

By way of exemplification, cationic polymeric conditioning agents preferred for use herein include cationic guar gums such as hydroxypropyl trimethyl ammonium guar gum (d.s. of from 0.11 to 0.22) available commercially under the trade names Jaguar C-14-S(RTM) and Jaguar C-17(RTM), and also Jaguar C-16(RTM), which contains hydroxypropyl substituents (d.s. of from 0.8 - 1.1) in addition to the above-specified cationic groups, and quaternized cellulose esters available commercially under the trade names Ucare Polymer JR and Celquat. Other suitable cationic polymers are homopolymers of dimethyldiallylammonium chloride available commercially under the trade name Merquat 100, copolymers of dimethyl aminoethylmethacrylate and acrylamide, copolymers of dimethyldiallylammonium chloride and acrylamide, available commercially under the trade names Merquat 550 and Merquat S, quaternized vinyl pyrrollidone acrylate or methacrylate copolymers of amino alcohol available commercially under the trade name Gafquat, and polyalkylenimines such as polyethylenimime and ethoxylated polyethylenimine.

A number of additional optional materials can be added to the compositions of the invention. Such materials include keratolytic agents such as salicylic acid; proteins and polypeptides and derivatives thereof; water-soluble or solubilizable preservatives such as Germall 115, methyl, ethyl, propyl and butyl esters of hydroxybenzoic acid, EDTA, Euxyl (RTM) K400, Bronopol (2-bromo-2-nitropropane-1, 3-diol); antibacterials such as Irgasan (RTM), phenoxyethanol and phenoxypropanol (preferably at levels of from about 0.2% to about 5%); other moisturizing agents such as hyaluronic acid, chitin, and starch-grafted sodium polyacrylates such as Sanwet (RTMO IM-1000, IM-1500 and IM-2500 available from Celanese Superabsorbent Materials, Portsmith. VA, USA and described in USA-A-4,076,663; colouring agents; Pearling agents; perfumes and perfume solubilizers etc. Water is also present at a level of from about 50% to about 98.9%, preferably from about 70% to about 95% by weight of the compositions herein.

The pH of the compositions is preferably from about 4 to about 9, more preferably from about 4.5 to about 7.

The following examples further describe and demonstrate embodiments within the scope of the present invention. The examples are given solely for the purpose of illustration and are not to be construed as limitations of the present invention.

Ingredients are identified by chemical or CTFA name.

### Example I

A facial cleansing composition of the present invention is made as follows:

| Ingredient | % w/w |
|---|---|
| Phase A | |
| Water | q.s. |
| Polyquaternium -10 | 0.50 |

| Phase B | |
|---|---|
| Potassium Coco Hydrolyzed Animal Protein² | 15.0 |
| Amphoteric Surfactant I³ | 5.1 |
| Amphoteric Surfactant II⁴ | 6.8 |
| Amphoteric Surfactant III⁵ | 7.3 |

| Phase C | |
|---|---|
| Glycerin | 3.0 |
| Polyol Alkoxy Ester⁶ | 1.6 |
| PEG-120 Methyl Glucose Dioleate⁷ | 0.6 |

| Phase D | |
|---|---|
| Preservative (phenoxyethanol) | 0.4 |

| Phase E | |
|---|---|
| Water | 1.0 |
| Na₄EDTA | 0.1 |

| | |
|---|---|
| ¹ Available as Polymer JR 400 | |
| ² Available as Lamepon S | |
| ³ Cocoamphocarboxyglycinate (and) Na Lauryl Sulfate (and) Hexylene Glycol available as Miranol 2MCA MOD | |
| ⁴ Na Lauryl Sarcosinate available as Hamposyl- L-30 | |
| ⁵ Na Lauriminodipropionate available as Mirataine H2C-HA | |
| ⁶ Available as Crothix | |
| ⁷ Available as Glucamate DOE-120 | |

The water is heated to 65°C and the polyquaternium-10 is added to the water to form Phase A. The Phase B ingredients are added sequentially to this phase. Separately, the Phase C components are heated to 65°C. Phase C is combined with this mixture and then cooled to 40°C. Phase D and Phase E are added to this mix to form the resultant cosmetic composition.

Application of approximately 2 grams of the resulting gel/viscous liquid with water is useful for topical application as a cleanser to remove, for example, dirt and oil as well as difficult to remove make-up, waterproof mascara and the like.

### Example II

A facial cleansing composition of the present invention is made by combining the following ingredients utilizing conventional mixing techniques as described above in Example I.

| Ingredient | % w/w |
|---|---|
| Phase A | |
| Water | q.s. |
| Polyquaternium -10¹ | 0.50 |

| Phase B | |
|---|---|
| Amphoteric Surfactant I² | 15.1 |
| Amphoteric Surfactant II³ | 4.0 |

| Phase C | |
|---|---|
| Glycerin | 3.0 |
| Polyol Alkoxy Ester⁴ | 1.6 |
| PEG-120 Methyl Glucose Dioleate⁵ | 0.6 |

| Phase D | |
|---|---|
| Preservative (phenoxyethanol) | 0.4 |

| Phase E | |
|---|---|
| Water | 1.0 |
| Na₄EDTA | 0.1 |

| | |
|---|---|
| ¹ Available as Polymer JR 400 | |
| ² Na Lauriminodipropionate available as Mirataine H2C-HA | |
| ³ Cocoamphocarboxyglycinate (and) Na Lauryl Sulfate (and) Hexylene Glycol available as Miranol 2MCA MOD | |
| ⁴ Available as Crothix | |
| ⁵ Available as Glucamate DOE-120 | |

Application of approximately 2 grams of the resulting gel/viscous liquid with water is useful for topical application as a cleanser to remove, for example, dirt and oil as well as difficult to remove make-up, waterproof mascara and the like.

### Example III

A facial cleansing composition of the present invention is made by combining the following ingredients utilizing conventional mixing techniques as described above in Example I.

| Ingredient | % w/w |
|---|---|
| Phase A | |
| Water | q.s. |
| Polyquaternium -10¹ | 0.50 |

| Phase B | |
|---|---|
| Amphoteric Surfactant I² | 15.1 |
| Amphoteric Surfactant II³ | 4.0 |

| Phase C | |
|---|---|
| Glycerin | 3.0 |
| Polyol Alkoxy Ester⁴ | 1.0 |
| Na Acrylate/Stearate 20 Methacrylate polymer⁵ | 3.0 |

| Phase D | |
|---|---|
| Preservative | 0.4 |

| Phase E | |
|---|---|
| Water | 1.0 |
| Na₄EDTA | 0.1 |

| | |
|---|---|
| ¹ Available as Polymer JR 400 | |
| ² Na Lauriminodipropionate available as Deriphat 160C | |
| ³ Cocoamphocarboxyglycinate (and) Na Lauryl Sulfate (and) Hexylene Glycol available as Miranol 2MCA MOD | |
| ⁴ Available as Crothix | |
| ⁵ Available as Aculyn 22 | |

Application of approximately 2 grams of the resulting gel/viscous liquid with water is useful for topical application as a cleanser to remove, for example, dirt and oil as well as difficult to remove make-up, waterproof mascara and the like.

### Example IV

A facial cleansing composition of the present invention is made by combining the following ingredients utilizing conventional mixing techniques as described above in Example I.

| Ingredient | % w/w |
|---|---|
| Phase A | |
| Water | q.s. |
| Polyquaternium -10¹ | 0.50 |

| Phase B | |
|---|---|
| Amphoteric Surfactant I² | 8.0 |
| Amphoteric Surfactant II³ | 8.0 |

| Phase C | |
|---|---|
| Glycerin | 15.0 |
| Polyol Alkoxy Ester⁴ | 1.5 |
| PEG-120 Methyl Glucose Dioleate⁵ | 0.5 |

| Phase D | |
|---|---|
| Preservative (phenoxyethanol) | 0.4 |

| Phase E | |
|---|---|
| Water | 1.0 |
| Na₄EDTA | 0.1 |

| | |
|---|---|
| ¹ Available as Polymer JR 400 | |
| ² Lauroamphodiacetate (and) Na Trideceth Sulfate available as Miranol BT | |
| ³ Na Lauriminodipropionate available as Mirataine H2C-HA | |
| ⁴ Available as Crothix | |
| ⁵ Available as Glucomate DOE-120 | |

Application of approximately 2 grams of the resulting gel/viscous liquid with water is useful for topical application as a cleanser to remove, for example, dirt and oil as well as difficult to remove make-up, waterproof mascara and the like.

## Claims

1. A foaming cosmetic cleansing composition comprising;
(a) from about 0.1% to about 5.0% by weight on a solids basis of an amphoteric surfactant selected from imidazolinium derivatives of formula I 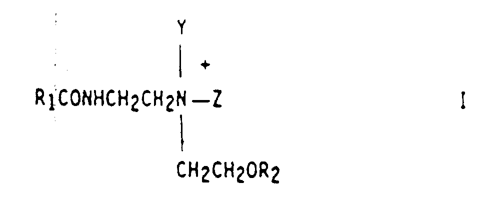 wherein R₁ is C₈-C₂₂ alkyl or alkenyl, R₂ is hydrogen or CH₂COOM, Z is H, CH₂COOM, CH₂CH₂COOM, or CH₂CHOHCH₂SO₃M, Y is H, CH₂COOM, CH₂CH₂COOM, or CH₂CHOHCH₂SO₃M and M is H, alkali metal, alkaline earth metal, ammonium or alkanolammonium;
(b) from about 0.1% to about 5.0% of a polyol alkoxy ester which is a branched polyethylene-glycol compound having molecular weight about 4000 to about 8000; and
(c) from about 60% to about 99.5% water;
wherein the ratio of (a):(b) is from about 15:1 to about 1:1 and wherein the cleansing composition has a viscosity of at least about 150 cps (Brookfield RVT, Spindle No TB, 10 rpm, 25°C).

2. A cosmetic composition according to Claim 1 which further comprises from about 0.5% to about 16% by weight of a second amphoteric surfactant selected from aminoalkanoates of formula II
R₁NH(CH₂)ₙCO₂M II
iminodialkanoates of formula III
R₁N[(CH₂)ₘCO₂M]₂ III
and mixtures thereof, wherein R₁ is C₈-C₂₂ alkyl or alkenyl, R₂ is hydrogen or CH₂CH₂M, Z is CO₂M or CH₂CO₂M and M is H, alkali metal, alkaline earth metal, ammonium or alkanolammonium; and wherein n and m are numbers from 1 to 4, and wherein the mixture of first and second amphoteric surfactants comprises at least about 30% by weight of the total surfactant component.

3. A cosmetic composition according to Claim 2 which comprises from about 0.5% to about 2.0% of the polyol alkoxy ester and wherein the total surfactant concentration is from about 1% to about 20% by weight of the cleansing composition.

4. A cosmetic composition according to Claim 3 wherein the ratio of amphoteric surfactant:polyol alkoxy ester is from about 10:1 to about 1:3 and further comprises from about 1% to about 10% by weight of each of the first and second amphoteric surfactants.

5. A cosmetic composition according to Claim 4 wherein the weight ratio of first amphoteric surfactant:second amphoteric surfactant is from about 10:1 to about 1:10.

6. A cosmetic composition according to Claim 5 wherein the weight ratio of first amphoteric surfactant:second amphoteric surfactant is from about 3:1 to about 1:3, and wherein the composition additionally comprises from about 0.1% to about 10%, by weight of anionic surfactant.

7. A cosmetic composition according to Claim 6 wherein the ratio of amphoteric surfactant:polyol alkoxy ester is from about 3:1 to about 1:3 and comprises a premix or complex of the first amphoteric surfactant and anionic surfactant in an equivalent ratio of about 1:1.

8. A cosmetic composition according to claim 7 wherein the anionic surfactant is a fatty acid condensation product selected from
(i) a condensation product of C₈-C₁₂, preferably C₁₀-C₁₈ fatty acids with hydrolysed proteins,
(ii) fatty acid sarcosinates derived from C₈-C₂₂, preferably C₁₀-C₁₈ fatty acids, and
(iii) mixtures thereof.

9. A cosmetic composition according to Claim 8 having a viscosity of from about 150 to about 20,000 cps (Brookfield RVT, Spindle No. TB, 10 rpm, 25°C).

10. A cosmetic composition according to Claim 9 wherein the aqueous cleansing composition comprises from about 1% to about 20% of a hair or skin moisturizer.

11. A cosmetic composition according to Claim 10 wherein the moisturiser is nonocclusive and is selected from:
(i) water-soluble liquid polyols;
(ii) essential amino acid compounds found naturally occuring in the stratum corneum of the skin; and
(iii) water-soluble nonpolyol nonocclusives and mixtures thereof.

12. A cosmetic composition according to Claim 11 wherein the moisturiser is selected from glycerin, polyethylene glycol, propylene glycol. sorbitol, polyethylene glycol and propylene glycol esters of methyl glucose, polyethylene glycol and propylene glycol esters of lanolin alcohol, sodium pyrrolidone carboxylic acid, lactic acid, L-proline and mixtures thereof.

13. A cosmetic composition according to Claim 12 wherein the moisturiser is glycerin.

14. A cosmetic composition according to Claim 13 additionally comprising from abut 0.05% to about 5% by weight of a cationic or nonionic polymeric hair or skin conditioning agent.

15. A cosmetic composition according to Claim 14 wherein the polymeric hair or skin conditioning agent is selected from cationic and nonionic polysacharides; cationic and nonionic homopolymers and copolymers derived from acrylic and/or methacrylic acid; cationic and nonionic cellulose resins; cationic copolymers of dimethyldiallylammonium chloride and acrylic acid; cationic homopolymers of dimethyldiallylammonium chloride; cationic polyalkylene and ethoxypolyalkylene imines, and mixtures thereof.

## Patentansprüche

1. Schaumbildende kosmetische Reinigungszusammensetzung, umfassend:
(a) etwa 0,1 bis etwa 5,0 Gew.-%, auf Feststoffbasis, eines amphoteren Tensids, gewählt aus Imidazoliniumderivaten der Formel I 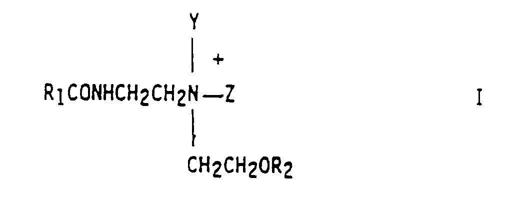 worin R₁ ein C₈-C₂₂-Alkyl oder -Alkenyl ist, R₂ Wasserstoff oder CH₂COOM ist, Z H, CH₂COOM, CH₂CH₂COOM, oder CH₂CHOHCH₂SO₃M ist, Y H, CH₂COOM, CH₂CH₂COOM oder CH₂CHOHCH₂SO₃M ist, worin M H, Alkalimetall, Erdalkalimetall, Ammonium oder Alkanolammonium ist;
(b) etwa 0,1 bis 5,0% eines Polyolalkoxyesters, bei dem es sich um eine verzweigte Polyethylenglykolverbindung mit einem Molekulargewicht von etwa 4000 bis etwa 8000 handelt; und
(c) etwa 60 bis etwa 99,5% Wasser;
wobei das Verhältnis von (a):(b) etwa 15:1 bis etwa 1:1 beträgt, und wobei die Reinigungszusammensetzung eine Viskosität von mindestens etwa 150 cps (Brookfield RVT, Spindel Nr. TB, 10 U/min, 25°C) besitzt.

2. Kosmetische Zusammensetzung nach Anspruch 1, umfassend weiterhin etwa 0,5 bis etwa 16 Gew.-% eines zweiten amphoteren Tensids, gewählt aus Aminoalkanoaten der Formel II
R₁NH(CH₂)ₙCO₂M
Iminodialkanoaten der Formel III
R₁N[(CH₂)ₘCO₂M]₂ III
und Mischungen davon, worin R₁ ein C₈-C₂₂-Alkyl oder -Alkenyl ist, R₂ Wasserstoff oder CH₂CH₂M ist, Z CO₂M oder CH₂CO₂M ist und wobei M H, Alkalimetall, Erdalkalimetall, Ammonium oder Alkanolammonium ist; und worin n und m Zahlen von 1 bis 4 sind, und wobei die Mischung aus dem ersten und zweiten amphoteren Tensid mindestens etwa 30 Gew.-% der gesamten Tensidkomponente ausmacht.

3. Kosmetische Zusammensetzung nach Anspruch 2, umfassend etwa 0,5 bis etwa 2,0 % des Polyolalkoxyesters, wobei die Gesamttensidkonzentration etwa 1 bis etwa 20 Gew.-% der Reinigungszusammensetzung beträgt.

4. Kosmetische Zusammensetzung nach Anspruch 3, wobei das Verhältnis von amphoterem Tensid:Polyolalkoxyester etwa 10:1 bis etwa 1:3 beträgt, und umfassend weiterhin etwa 1 bis etwa 10 Gew.-% an jeweils dem ersten und zweiten amphoteren Tensid.

5. Kosmetische Zusammensetzung nach Anspruch 4, wobei das Gewichtsverhältnis von erstem amphoteren Tensid: zweitem amphoteren Tensid etwa 10:1 bis etwa 1:10 beträgt.

6. Kosmetische Zusammensetzung nach Anspruch 5, wobei das Gewichtsverhältnis von erstem amphoteren Tensid: zweitem amphoteren Tensid etwa 3:1 bis etwa 1:3 beträgt und wobei die Zusammensetzung weiterhin etwa 0,1 bis etwa 10 Gew.-% eines anionischen Tensids umfaßt.

7. Kosmetische Zusammensetzung nach Anspruch 6, wobei das Verhältnis von amphoterem Tensid: Polyolalkoxyester etwa 3:1 bis etwa 1:3 beträgt, und umfassend eine Vormischung oder einen Komplex aus dem ersten amphoteren Tensid und einem anionischen Tensid in einem Äquivalentverhältnis von etwa 1:1.

8. Kosmetische Zusammensetzung nach Anspruch 7, wobei das anionische Tensid ein Fettsäurekondensationsprodukt ist, welches aus
(i) einem Kondensationsprodukt aus C₈-C₁₂-, vorzugsweise C₁₀-C₁₈-Fettsäuren mit hydrolysierten Proteinen,
(ii) Fettsäuresarcosinaten, welche von C₈-C₂₂-, vorzugsweise C₁₀-C₁₈-Fettsäuresäuren abgeleitet sind, und
(iii) Mischungen davon
gewählt ist.

9. Kosmetische Zusammensetzung nach Anspruch 8, welche eine Viskosität von etwa 150 bis etwa 20000 cps (Brookfield RVT, Spindel Nr. TB, 10 U/min, 25°C) besitzt.

10. Kosmetische Zusammensetzung nach Anspruch 9, wobei die wäßrige Reinigungszusammensetzung etwa 1 % bis etwa 20 % eines Haar- oder Haut-Feuchthaltemittels umfaßt.

11. Kosmetische Zusammensetzung nach Anspruch 10, wobei das Feuchthaltemittel nichtokklusiv und aus:
(i) wasserlöslichen flüssigen Polyolen;
(ii) essentiellen Aminosäureverbindungen, welche natürlich vorkommen und im Stratum corneum der Haut auftreten; und
(iii) wasserlöslichen Nichtpolyol-Nichtokklusiven und Mischungen davon gewählt ist.

12. Kosmetische Zusammensetzung nach Anspruch 11, wobei das Feuchthaltemittel aus Glyzerin, Polyethylenglykol, Propylenglykol, Sorbit, Polyethylenglykol- und Propylenglykolestern von Methylglukose, Polyethylenglykol- und Propylenglykolestern von Lanolinalkohol, Natriumpyrrolidoncarbonsäure, Milchsäure, L-Prolin und Mischungen davon gewählt ist.

13. Kosmetische Zusammensetzung nach Anspruch 12, wobei das Feuchthaltemittel Glyzerin ist.

14. Kosmetische Zusammensetzung nach Anspruch 13, umfassend weiterhin etwa 0,05 bis etwa 5 Gew.-% eines kationischen oder nichtionischen, polymeren Haar- oder Hautkonditioniermittels.

15. Kosmetische Zusammensetzung nach Anspruch 14, wobei das polymere Haar- oder Haut-Konditioniermittel aus kationischen und nichtionischen Polysacchariden; kationischen und nichtionischen Homopolymeren und Copolymeren, welche von Acryl- und/oder Methacrylsäure abgeleitet sind; kationischen und nichtionischen Celluloseharzen; kationischen Copolymeren von Dimethyldiallylammoniumchlorid und Acylsäure; kationischen Homopolymeren von Dimethyldiallylammoniumchlorid; kationischen Polyalkylen- und Ethoxypolyalkyleniminen und Mischungen davon gewählt ist.

## Revendications

1. Composition cosmétique nettoyante moussante, comprenant:
(a) d'environ 0,1 % à environ 5,0 % en poids, sur la base des matières solides, d'un tensioactif amphotère choisi parmi les dérivés d'imidazolinium de formule I 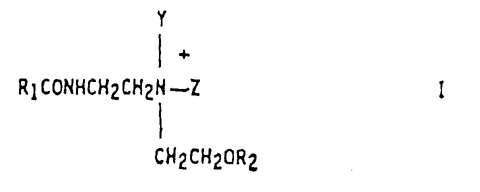 dans laquelle R₁ est un groupe alkyle ou alcényle en C₈-C₂₂, R₂ est un atome d'hydrogène ou un groupe CH₂COOM, Z est H, CH₂COOM, CH₂CH₂COOM ou CH₂CHOHCH₂SO₃M, Y est H, CH₂COOM, CH₂CH₂COOM ou CH₂CHOHCH₂SO₃M, et M est H, un métal alcalin, un métal alcalino-terreux, un ammonium ou un alcanolammonium;
(b) d'environ 0,1 % à environ 5,0 % d'un alcoxyester de polyol qui est un composé polyéthylèneglycol ramifié ayant une masse moléculaire d'environ 4000 à environ 8000; et
(c) d'environ 60 % à environ 99,5 % d'eau ;
dans laquelle le rapport (a):(b) est d'environ 15:1 à environ 1:1, et dans laquelle la composition nettoyante a une viscosité d'au moins environ 150 cP (RVT Brookfield, broche n° TB, 10 tours/min, 25°C).

2. Composition cosmétique selon la revendication 1, qui comprend aussi d'environ 0,5 % à environ 16 % en poids d'un second tensioactif amphotère choisi parmi les aminoalcanoates de formule II
R₁NH(CH₂)ₙCO₂M II
les iminodialcanoates de formule III
R₁N[(CH₂)ₘCO₂M]₂ III
et des mélanges de ceux-ci, dans lesquels R₁ est un groupe alkyle ou alcényle en C₈-C₂₂, R₂ est un atome d'hydrogène ou un groupe CH₂CH₂M, Z est CO₂M ou CH₂CO₂M et M est H, un métal alcalin, un métal alcalino-terreux, un ammonium ou un alcanol-ammonium; et dans lesquels n et m sont des nombres de 1 à 4, et dans lesquels le mélange des premier et second tensioactifs amphotères constitue au moins environ 30% en poids du constituant tensioactif total.

3. Composition cosmétique selon la revendication 2, qui comprend d'environ 0,5% à environ 2,0% de l'alcoxyester de polyol et dans laquelle la concentration totale en tensioactif est d'environ 1% à environ 20% en poids de la composition nettoyante.

4. Composition cosmétique selon la revendication 3, dans laquelle le rapport tensioactif amphotère/alcoxyester de polyol est d'environ 10:1 à environ 1:3 et qui comprend aussi d'environ 1% à environ 10% en poids de chacun des premier et second tensioactifs amphotères.

5. Composition cosmétique selon la revendication 4, dans laquelle le rapport pondéral premier tensioactif amphotère/second tensioactif amphotère est d'environ 10:1 à environ 1:10.

6. Composition cosmétique selon la revendication 5, dans laquelle le rapport pondéral premier tensioactif amphotère/second tensioactif amphotère est d'environ 3:1 à environ 1:3, et dans laquelle la composition comprend aussi d'environ 0,1% à environ 10%, en poids, de tensioactif anionique.

7. Composition cosmétique selon la revendication 6, dans laquelle le rapport tensioactif amphotère/alcoxyester de polyol est d'environ 3:1 à environ 1:3 et qui comprend un prémélange ou complexe du premier tensioactif amphotère et d'un tensioactif anionique, dans un rapport d'equivalents d'environ 1:1.

8. Composition cosmétique selon la revendication 7, dans laquelle le tensioactif anionique est un produit de condensation d'acide gras choisi parmi:
(i) un produit de condensation d'acides gras en C₈-C₂₂, de préférence en C₁₀-C₁₈, avec des protéines hydrolysées,
(ii) des sarcosinates d'acides gras dérivés d'acides gras en C₈-C₂₂, de préférence en C₁₀-C₁₈, et
(iii) des mélanges de ceux-ci.

9. Composition cosmétique selon la revendication 8, ayant une viscosité d'environ 150 à environ 20 000 cP (RVT Brookfield, broche n° TB, 10 tours/min, 25°C).

10. Composition cosmétique selon la revendication 9, dans laquelle la composition nettoyante aqueuse comprend d'environ 1% à environ 20% d'un hydratant pour les cheveux ou pour la peau.

11. Composition cosmétique selon la revendication 10, dans laquelle l'hydratant est un produit non occlusif choisi parmi:
(i) les polyols liquides hydrosolubles;
(ii) les composés acides aminés essentiels que l'on trouve à l'état naturel dans la couche cornée de la peau; et
(iii) les produits non occlusifs non polyols hydrosolubles, et des mélanges de ceux-ci.

12. Composition cosmétique selon la revendication 11, dans laquelle l'hydratant est choisi parmi la glycérine, le polyéthylèneglycol, le propylèneglycol, le sorbitol, les esters de polyéthylèneglycol et de propylèneglycol de méthylglucose, les esters de polyéthylèneglycol et de propylèneglycol d'alcool de lanoline, le pyrrolidonecarboxylate de sodium, l'acide lactique, la L-proline et des mélanges de ceux-ci.

13. Composition cosmétique selon la revendication 12, dans laquelle l'hydratant est la glycérine.

14. Composition cosmétique selon la revendication 13, comprenant aussi d'environ 0,05% à environ 5% en poids d'un agent polymère cationique ou non ionique de traitement des cheveux ou de la peau.

15. Composition cosmétique selon la revendication 14, dans laquelle l'agent polymère de traitement des cheveux ou de la peau est choisi parmi les polysaccharides cationiques et non ioniques; les homopolymères et les copolymères cationiques et non ioniques dérivés de l'acide acrylique et/ou méthacrylique; les résines de cellulose cationiques et non ioniques; les copolymères cationiques de chlorure de diméthyldiallylammonium et d'acide acrylique; les homopolymères cationiques de chlorure de diméthyldiallylammonium; les polyalkylène- et éthoxypolyalkylène-imines cationiques; et des mélanges de ceux-ci.
